# EUROPÄISCHE PATENTSCHRIFT

(11) **EP 1 418 427 B1**
(45) Veröffentlichungstag und Bekanntmachung des Hinweises auf die Patenterteilung: **27.06.2012**
(21) Anmeldenummer: 03022353.1
(22) Anmeldetag: 04.10.2003
(51) Int. Cl.: G01N 33/487, G01N 33/50, C12Q 1/02, C12M 1/34

(54) **Vorrichtung und Verfahren zur Messung elektrischer Vorgänge an biologischen Membranen**
Apparatus and method for measuring electrical processes in biological membranes
Appareil et procédé de mesure de processus éléctriques en membranes biologiques

(30) Priorität: 07.11.2002 DE 10251767
(43) Veröffentlichungstag der Anmeldung: 12.05.2004
(73) Patentinhaber: Forschungszentrum Jülich GmbH, 52425 Jülich (DE)
(72) Erfinder: Lühring, Hinrich, Dr., 52428 Jülich (DE)

(56) Entgegenhaltungen:
- WO-A-01/59447
- WO-A-02/04943
- WO-A-02/20087
- WO-A-99/66329
- KOURIE J I: "Significance of potassium and chloride membrane currents in mechanisms of salt tolerance in Chara corallina and Chara inflata" DESALINATION, ELSEVIER SCIENTIFIC PUBLISHING CO, AMSTERDAM, NL, Bd. 106, Nr. 1, 1. August 1996 (1996-08-01), Seiten 431-434, XP004019197 ISSN: 0011-9164

## Beschreibung

Die Erfindung betrifft eine Vorrichtung sowie ein Verfahren zur Messung elektrischer Vorgänge an biologischen Membranen.

Mit Hilfe von Membranen stellen lebende Zellen in ihrem Inneren eine Mikroumgebung her, die die Funktion des zellulären Apparates sichert. Biologische Membranen sind selektiv permeabel für viele polare Moleküle. Mit Hilfe spezifischer Transportproteine ist es möglich, Moleküle durch die Membran zu schleusen. Dies ist immer dann der Fall, wenn die Zelle lebenswichtige Nährstoffe aufnimmt oder Metaboliten ausscheidet. Außerdem muß die Zelle die intrazellulären Ionenkonzentrationen genau regulieren und daher bestimmte Ionen aufnehmen oder abgeben. Der ionische Ursprung der Membranspannung wurde zuerst von Loeb (J. Loeb 1897: The physiological problems of today. In: J. Loeb:Studies in General Physiology, University of Chicago Press, Chicago, 1905, pp. 497-500; J. Loeb 1900: On ion-proteid compounds and their role in the mechanics of life phenomena, I. The poisonous character of a pure NaCl solution. Am. J. Physiol. 3: 327-338) diskutiert. Später zeigte sich, daß die Membranspannung durch aktive Transportsysteme erzeugt und hauptsächlich durch den transmembranalen K⁺-Konzentrationsgradienten moduliert wird. Im Ruhezustand ist die Plasmamembran für K⁺ durchlässiger als für Na⁺ und Anionen. Der K⁺-Gradient und die Unterschiede in den Permeabilitäten für verschiedene Ionen werden dabei durch die Eigenschaften spezifischer Transportproteine in der Plasmamembran hervorgerufen. In tierischen Zellen sind insbesondere zwei spezifische Transportproteine für die Elektrogenese der Membran verantwortlich: Die sogenannte Na⁺-K⁺-Pumpe tauscht aktiv intrazelluläres Na⁺ gegen extrazelluläres K⁺ aus, so daß die intrazelluläre K⁺-Konzentration höher ist als außerhalb der Zelle. Für Na⁺ gilt das Gegenteil. Das andere Transportprotein ist ein K⁺-drain-Kanal, der die K⁺-Ionen entlang ihres Konzentrationsgradienten die Membran ins extrazelluläre Milieu passieren läßt. Der Verlust positiver Ladungen im Zellinneren führt zum Erreichen einer negativen Membranspannung. Ein Gleichgewichtszustand ist dann erreicht, wenn elektrische und osmotische Arbeit - die passiven Triebkräfte des Ionenflusses - gleich sind. Ähnliche Transportphänomene bestimmen den elektrischen Zustand der pflanzlichen Membranen. Hier beruht die Elektrogenese vor allem auf dem aktiven Transport einer H⁺-ATPase, die Protonen aus dem Cytosol ins extrazelluläre Medium pumpt. Sekundäre Transportsysteme (Ionenkanäle etc.) beziehen ihre Triebkraft aus dem erzeugten elektrochemischen Gradienten.

Die Membranspannung pflanzlicher Zellen liegt im Bereich von ca. -200 mV. Insbesondere Charophyten wurden auf Grund ihrer einfachen Geometrie und ungewöhnlichen Zellgröße als favorisierte Untersuchungsobjekte in der Membranforschung benutzt. Ihre Zellen erreichen Längen von einigen Zentimetern bei Durchmessern von etwa 1 mm. Die Plasmamembran der Charophyten ist elektrisch erregbar, das heißt, als Folge einer Depolarisation um ca. 30 mV wird ein sogenanntes Aktionspotential ausgelöst, das im Maximum etwa 0 mV erreicht. Depolarisation und Repolarisation werden durch zeitlich aufeinanderfolgende transiente Flüsse von Cl- und K⁺ aus der Zelle verursacht.

Im allgemeinen werden Spannungsmessungen an intakten Zellen folgendermaßen vorgenommen: Eine Zelle befindet sich in einer Meßkammer, die mit einer adäquaten Salzlösung gefüllt ist. In die Lösung der Kammer wird eine Referenzelektrode getaucht (Ag/AgCl). Eine mit Salzlösung gefüllte Glasmikropipette wird mit Hilfe eines Mikromanipulators in die Zelle eingestochen. Zur Ableitung des intrazellulären Potentials dient eine Ag/AgCl-Elektrode, die sich in der Glasmikropipette in Kontakt mit der Salzlösung befindet. Die Potentialdifferenz zwischen Referenz- und Glasmikroelektrode ist identisch mit der Membranspannung. Diese Meßanordnung wird sowohl für tierische als auch pflanzliche Zellen eingesetzt. Zur weitergehenden Charakterisierung der elektrischen Membraneigenschaften wird über eine zweite eingestochene Glasmikroelektrode ein Strom injiziert, mit dessen Hilfe der Membranwiderstand bestimmt werden kann.

In der Arbeit von Shimmen et al. (T. Shimmen, M. Kikuyama, M. Tazawa 1976: Demonstration of two stable potential states of plasmalemma of Chara without tonoplast. J. Membrane Biol. 30: 249-270)) wurde eine große Internodialzelle von Chara durch Vaselinebrücken in drei Abschnitte partitioniert und in einer Meßkammer mit drei Kompartimenten angeordnet. Hier konnte auf eine inserierte strominjizierende Glasmikroelektrode verzichtet werden, da der Strom an den beiden Zellenden in den äußeren Kammerkompartimenten über extrazelluläre Elektroden appliziert wurde und erst im mittleren Kompartiment der Meßkammer über die Membran zur Erdelektrode fließen konnte. Eine Glasmikroelektrode im zentralen Meßkammerkompartiment leitete das intrazelluläre Potential ab, Spannungsmeßkreis und Stromkreis waren voneinander unabhängig.

In derselben Arbeit stellten die Autoren eine Meßtechnik, die sogenannte "K⁺-Anästhesie-Methode" vor, bei deren Anwendung auf die Insertion von Glasmikroelektroden vollständig verzichtet wurde. In dieser Anordnung, die eine Meßkammer mit zwei Kompartimenten umfaßt, wurde ein Zellabschnitt der großen Chara- Internodialzelle in hoher KCl-Konzentration (ca. 100 mM) gehalten, so daß die Membranspannung in diesem Abschnitt auf 0 mV sank und der Membranwiderstand nur noch wenige 100 Ω betrug. Auf Grund dieser Wirkung kann diese KCl-Lösung auch als anästhetisierende Lösung bezeichnet werden. Die Membranspannung und der Membranwiderstand im anderen Zellabschnitt, der von künstlichem Teichwasser (0.1 mM Salzkonzentrationen) umgeben ist und nur mit einer adäquaten Konzentration von Sorbitol als Osmotikum ausbalanciert wird, bleiben erhalten (ca. -200 mV, 50-100 kΩ). Die ableitenden Elektroden befinden sich extrazellulär in den Kammerkompartimenten und leiten die Membranspannung ab. Diese Technik ist anwendbar, da die Elektrode im 100-mM-KCl-Kompartiment sich "virtuell" im Zellinnern befindet (0 mV Membranspannung) und somit das Einstechen der Elektrode in die Zelle überflüssig macht, während der Zellabschnitt im künstlichen Teichwasser unabhängig die Membranspannung der intakten Zelle aufweist.

Ein Nachteil der bisher bekannten Meßkammern und Meßverfahren liegt darin, daß bei Untersuchungen des Einflusses verschiedener Parameter auf das elektrische Verhalten der Membranen, für jeden Parameter neue Zellen präpariert und in die Versuchsanordnung gebracht werden müssen. An einer Membran können daher gleichzeitig nicht mehrere Parameter untersucht werden. Dies führt zum einen zu zeitaufwendigen Vorbereitungsmaßnahmen und zum anderen zu einer Verschlechterung der Vergleichbarkeit der Meßergebnisse, da die Reproduzierbarkeit der Experimente auf Grund unterschiedlicher Vitalität der Zellen nicht immer konstant gehalten werden kann.

Die WO01/59447A1 offenbart Mulit-well-platten, welche Kompartimente aufweisen, bei dem jedes Kompartiment eine Mehrzahl von Zellen aufnehmen kann. Die WO02/20087A1 zeigt ein Verfahren und eine Vorrichtung bei denen eine ganze Zelle mit Elektroden in Kontakt gebracht wird.

Es ist daher die Aufgabe der Erfindung, eine Vorrichtung und ein Verfahren zu schaffen, wodurch an einer biologischen Membran simultan der Einfluß unterschiedlicher Versuchsparameter auf das elektrische Verhalten der Zellmembran untersucht werden kann, bzw. Parallelversuche unter gleichen Bedingungen zur statistischen Absicherung der Ergebnisse durchgeführt werden können.

Ausgehend vom Oberbegriff des Anspruchs 1 wird die Aufgabe erfindungsgemäß gelöst mit den im kennzeichnenden Teil des Anspruchs 1 angegebenen Merkmalen. Weiterhin wird die Aufgabe ausgehend vom Oberbegriff des Anspruchs 8 erfindungsgemäß gelöst mit den im kennzeichnenden Teil des Anspruchs 8 angegebenen Merkmalen.

Mit der erfindungsgemäßen Vorrichtung und dem Verfahren ist es nunmehr möglich, an Membranflächen einer einzigen präparierten biologischen Membran simultan verschiedene Untersuchungen durchzuführen. Es ist weiterhin möglich, eine Vielzahl von Untersuchungen an einer Zelle gleichzeitig auf kleinstem Raum durchzuführen.

Vorteilhafte Weiterbildungen sind in den Unteransprüchen angegeben.

Die Zeichnungen zeigen eine beispielhafte Ausführungsform der erfindungsgemäßen Vorrichtung sowie experimentelle Ergebnisse des Verfahrens.

Es zeigt:
- Fig. 1:: Aufsicht der erfindungsgemäßen Vorrichtung mit der zugehörigen Meßeinrichtung
- Fig. 2:: Schnitt durch eine horizontal verlaufende Reihe X1 von Kompartimenten
- Fig. 3:: Schnitt durch eine vertikal verlaufende Reihe X1-X2-X3 von Kompartimenten
- Fig. 4:: Experimentelle Messung der Membranspannung und Bestimmung des Membranwiderstands durch Anwendung definierter Strompulse
- Fig. 5:: Auslösung eines Aktionspotentials durch mechanische Reizung
- Fig. 6:: Simultane Hemmung von Ionenkanälen, die für Depolarisation und Repolarisation während des Aktionspotentials verantwortlich sind

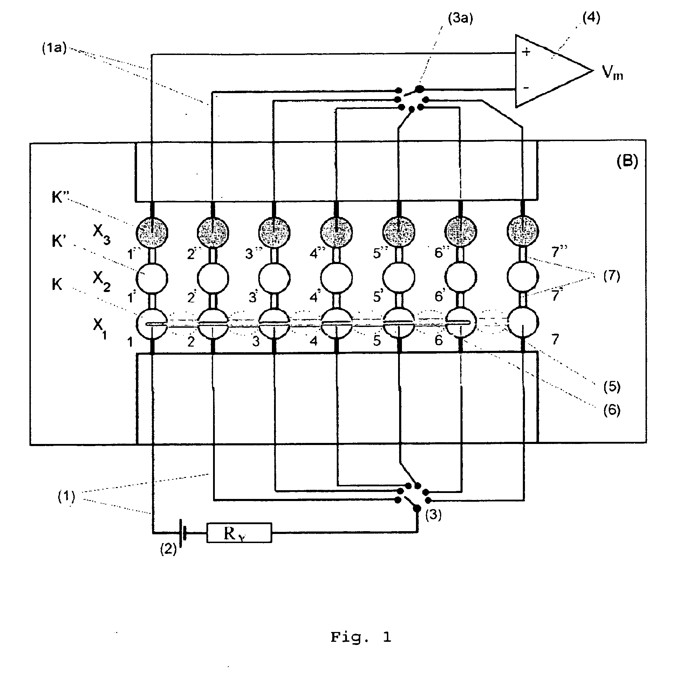

Figur 1 zeigt eine Aufsicht der erfindungsgemäßen Vorrichtung mit der zugehörigen Meßeinrichtung. Die Basisplatte (B) weist 21 kreisförmige Kompartimente (K, K', K") auf. Diese Kompartimente (K, K', K") sind gitterartig angeordnet: Es können drei horizontale Reihen mit je 7 Kompartimenten unterschieden werden: Reihe X1: Kompartimente 1 bis 7; Reihe X2: Kompartimente 1' bis 7' und Reihe X3: Kompartimente 1" bis 7". Sowohl die Kompartimente (K) der Reihe X1 als auch die Kompartimente (K") der Reihe X3 sind mit Bohrungen für das Einführen von Elektroden (1, 1a) versehen. Dabei sind die Elektroden (1), die mit den Kompartimenten (K) der Reihe X1 in Verbindung stehen, mit einer Stromquelle (2) verbunden. Zur Limitierung der Stromzufuhr können wählbare Vorwiderstände (Rv) eingesetzt werden. Durch einen Taster oder Schalter (3) wird der Stromkreis zwischen Kompartiment 1 und, je nach Stellung des Tasters (3), einem der Kompartimente 2 bis 7 geschlossen. Die Elektroden (1a), die mit den Kompartimenten aus Reihe X3 in Verbindung stehen, sind mit einem Verstärker und einem Spannungsmeßgerät (4) zur Detektion der Membranspannung versehen. Je nach Stellung des Tasters/Schalters (3a) kann die Membranspannung für einen Membranabschnitt in den Kompartimenten 2 bis 7 ermittelt werden. Die Kompartimente 1 bis 7 in X1 sind durch horizontale Kanäle (5) miteinander verbunden und dienen der Aufnahme der zu untersuchenden Zelle (6). Die Kompartimente 1-1'-1", 2-2'-2", 3-3'-3" usw. stehen jeweils über vertikale Kanäle (7) miteinander in Verbindung. Die Kompartimente werden mit folgenden unterschiedlichen Lösungen bzw. Substanzen gefüllt:
- Kompartiment 1:: 100 mM KCl
- Kompartimente 2 bis 7:: Künstliches Teichwasser
- Kompartimente 1'bis 7':: 2% w/v Agar in künstlichem Teichwasser
- Kompartimente 1" bis 7":: 1 M KCl

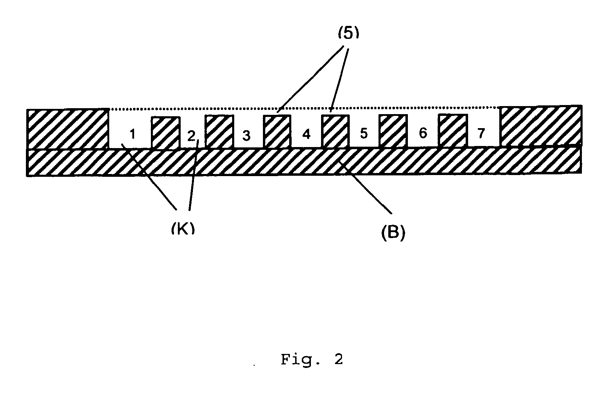

Figur 2 zeigt einen Schnitt durch die Kompartimente (K) der Reihe X1. Die in Figur 1 verwendeten Bezugszeichen sind entsprechend anzuwenden.

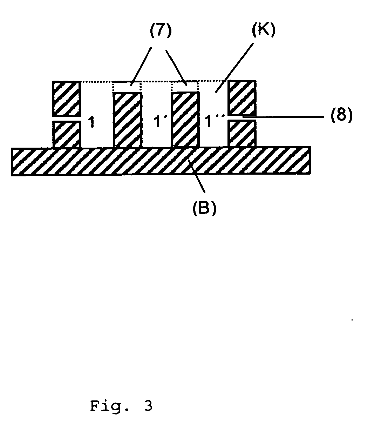

Figur 3 zeigt einen Schnitt durch einen der vertikal verlaufenden Kanäle (7) mit den Kompartimenten (K, K', K") wie beispielsweise durch die Kompartimente 1-1'-1". Die in Figur 1 verwendeten Bezugszeichen sind entsprechend anzuwenden. Die Elektroden (1, 1a) zum Anlegen des Stroms bzw. zum Ableiten des zu messenden Membranpotentials werden durch die Bohrungen (8) in die entsprechenden Kompartimente wie z. B. 1 und 1" geschoben.

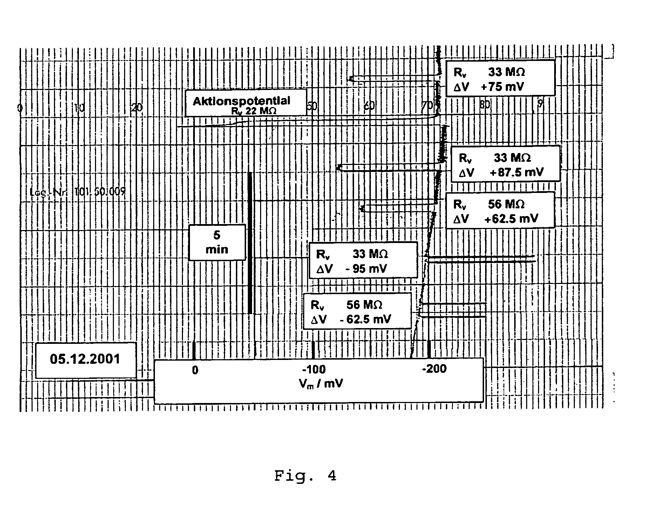

Fig. 4 zeigt Spannungsantworten der Zellmembran der Grünalge Chara corallina auf depolarisierende und hyperpolarisierende Ströme unter Einsatz der erfindungsgemäßen Vorrichtung. In der Meßkurve (Spannung als Funktion der Zeit) wurden die jeweils vorgeschalteten Widerstände Rᵥ in MΩ und die Auslenkung der Membranspannung ΔV in mV zu den entsprechenden Spannungsantworten eingefügt. Aus Spannungsantwort und dem vorgegebenen definierten Strom kann der Membranwiderstand berechnet werden. Der Versuch wurde in künstlichem Teichwasser durchgeführt. Der Strom wurde durch wählbare Vorwiderstände (Rᵥ in Fig. 1) über einen Bereich von 56-6.8 MΩ limitiert.

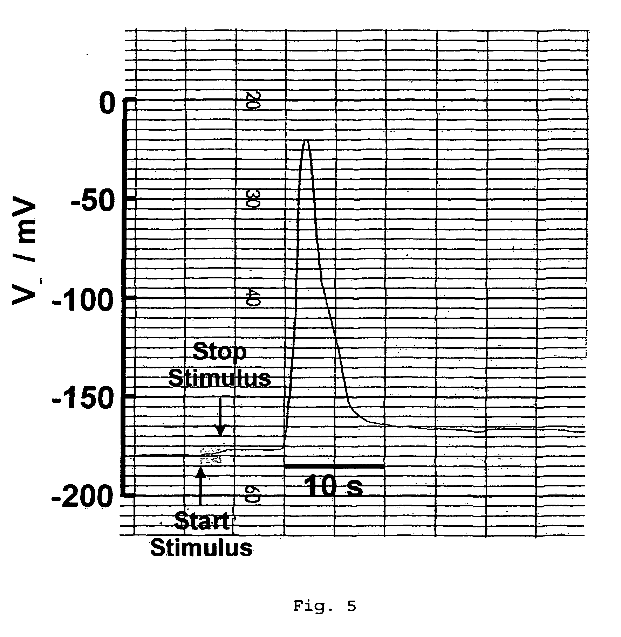

Fig. 5 zeigt ein Aktionspotential (Meßkurve: Spannung als Funktion der Zeit), das an der Membran von Chara australis durch einen mechanischen Stimulus ausgelöst wurde. Der Versuch wurde in künstlichem Teichwasser durchgeführt. Der zeitliche Verlauf des gemessenen Aktionspotentials ist identisch mit dem aus konventionell durchgeführten Experimenten (eingestochene Glasmikroelektrode).

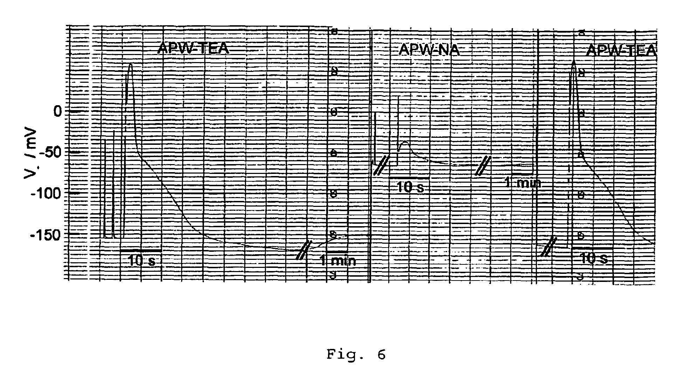

Fig. 6 zeigt das elektrische Verhalten einzelner Membranabschnitte einer Membran von Chara, die durch unterschiedliche Beladung der Kompartimente 2 und 3 unterschiedlichen Substanzen ausgesetzt wurden. Kompartiment 2 enthielt neben künstlichem Teichwasser zusätzlich 5 mM Tetraethylammoniumchlorid (TEA). Kompartiment 3 enthielt neben künstlichem Teichwasser 50 µM Nifluminsäure (NA). Im Verlauf des elektrisch hervorgerufenen Aktionspotentials in Kompartiment 2 (APW-TEA) erkennt man gegenüber dem in Figur 5 gemessenen Aktionspotential deutlich die verlangsamte Repolarisation. In Kompartiment 3 (APW-NA) unterband die Anwesenheit der Nifluminsäure die Entstehung eines Aktionspotentials nahezu komplett. Eine anschließende Messung in Kompartiment 2 (APW-TEA) ergab den identischen Verlauf des Aktionspotentials wie zu Beginn.

Im folgenden soll die erfindungsgemäße Vorrichtung und das Verfahren beispielhaft beschrieben werden.

Die vorliegende Erfindung betrifft eine Vorrichtung sowie ein Verfahren zur Messung elektrischer Vorgänge an biologischen Membranen. Mit Hilfe der erfindungsgemäßen Vorrichtung und dem Verfahren können an einer einzigen biologischen Membran die Einflüsse unterschiedlicher Parameter auf das elektrische Verhalten gleichzeitig untersucht werden. Unter biologischen Membranen sollen im Rahmen der vorliegenden Erfindungen sowohl pflanzliche als auch tierische Membranen sowie Gewebestreifen verstanden werden. Hierzu zählen beispielsweise große Pflanzenzellen, wie z. B. Zelle aus der Gruppe der Charophyten (Chara australis, Chara corallina) oder auch tierische Zellen wie z. B. Tintenfischaxone oder Zellen des Regenwurms (Bauchmark, Kolossalfasern).
Die erfindungsgemäße Vorrichtung umfaßt eine Basisplatte (B) mit mindestens 3 Kompartimenten (K), an die jeweils eine Elektrode (1) zur Stromzufuhr angeschlossen ist, und die Kompartimente (K) sowohl über einen Kanal (5) in einer Reihe X1 miteinander verbunden sind als auch jeweils über einen zweiten Kanal (7) mit weiteren Kompartimenten (K', K") verbunden sind, wobei die Kompartimente (K") jeweils an eine Elektrode (1a) zur Ableitung von Spannung angeschlossen sind. Die Basisplatte ist in einer vorteilhaften Ausführung des Verfahrens aus chemisch inertem Material wie z. B. Glas oder Plexiglas gefertigt und kann beispielsweise aus einer 20 mm starken Plexiglasplatte bestehen.
Zur Messung der elektrischen Vorgänge, d.h. Veränderungen der Membranspannung oder des Membranpotentials, an den biologischen Membranen, werden zunächst die Kompartimente (K) z. B. 1 bis 7 und (K") z. B. 1" bis 7" mit passenden Stopfen verschlossen. Anschließend werden die Kompartimente 1' bis 7' mit einer elektrisch leitenden aber diffusionshemmenden Substanz wie z. B. flüssigem 2% w/v Agar, der zuvor mit künstlichem Teichwasser aufgekocht wurde, gefüllt. Diese "Brücke" verhindert die Diffusion von Substanzen wie z. B. KCl-Lösungen zwischen den Kompartimenten K und K" entlang der Kanäle (7). Nach Gelierung des Agars werden die Stopfen entfernt und die Kanäle (5), welche die Kompartimente 1 bis 7 verbinden, mit einer elektrisch isolierenden Substanz wie z. B. Vaseline, Silikonöl o.ä. gefüllt. Darauf wird die zu untersuchende Zelle (6), wie z. B. eine Internodialzelle von Chara australis, entlang der Kompartimentreihe 1 bis 7 eingelegt und auf den Kanälen (5) zusätzlich mit Vaseline überschichtet. Auf diese Weise kann die Zelle (6) je nach Bedarf in unabhängig voneinander elektrisch reagierende Membranabschnitte unterteilt werden und ein extrazellulärer Kurzschluß zwischen den Kompartimenten (K) verhindert werden. Die Membranabschnitte stehen nur noch über das Zellinnere in Verbindung. Eine extrazelluläre Messung der elektrischen Reaktion der biologischen Membran ist nach der beschriebenen Unterteilung in Membranabschnitte nur noch im Bereich der Kompartimente (K) möglich.
Damit die eingelegte Zelle (6) möglichst wenig geschädigt wird, d. h. beispielsweise nicht austrocknet und inkrustiert, werden die Kompartimente 2 bis 7 möglichst schnell mit einer der Zelle physiologisch angepaßten Substanz wie z. B. bei Verwendung von Chara australis künstlichem Teichwasser, das mit ca. 180 mM Sorbitol osmotisch gegen 100 mM KCl ausgeglichen ist, gefüllt. Unter physiologisch angepaßten Substanzen werden Lösungen verstanden, die z. B. den osmotischen Bedingungen innerhalb der Zelle entsprechen und zu keiner Veränderung der natürlichen Physiologie der Zelle führen. Daraufhin wird Kompartiment 1 mit einer Substanz gefüllt, die zur Depolarisation der biologischen Membran in diesem Membranabschnitt führt, wie z. B. 100 mM KCl oder adäquate Ionophore. Diese Lösung wird auch als "anästhetisierende" Lösung bezeichnet. Zum Schluß werden die Kompartimente 1" bis 7" mit einer Substanz hoher Ionenstärke gefüllt, die das Entstehen unterschiedlicher Elektrodenpotentiale vermeidet, wie z. B. 1 M KCl. Die Elektroden (1a) in 1" und einem anderen " -Kompartiment werden an einen Verstärker mit einem Spannungsmeßgerät (4) angeschlossen.
Zur Messung der elektrischen Vorgänge d. h. Messung des Membranpotentials und des Membranwiderstands, wird unabhängig vom Spannungsmeßkreis über die Elektroden (1), z. B. Ag/AgCl-Elektroden, ein Strom appliziert, der in Kompartiment 1 in die Zelle eintritt und auf dem Weg über das Zellinnere, über mit Hilfe des Tasters/Schalters (3) wählbare Membranabschnitte der Kompartimente 2 bis 7, die Zelle verläßt. Durch adäquat gewählte Vorwiderstände (Rᵥ) kann der Strom definiert werden. Zur Stromapplikation kann beispielsweise eine 9-V-Batterie benutzt werden. Da die 100 mM KCl in Kompartiment 1 die Membran dieses Membranabschnitts auf 0 mV depolarisiert, befindet sich die Elektrode (1) des Kompartiments 1 dadurch "virtuell" im Zellinnern. Die Membranspannung (Vₘ) wird entlang der Kanäle (7) an die Kompartimente K" weitergeleitet in denen sich die Elektroden (1a) befinden und kann dann nach entsprechender Stellung des Schalters (3a) bestimmt werden. Soll beispielsweise die Membranspannung des Membranabschnitts in Kompartiment 2 bestimmt werden, so wird über die Elektrode (1) in Kompartiment 1 Strom in das Zellinnere wie oben beschrieben appliziert. Durch Einstellen des Schalters (3) in die Position, in der ein Meßkreis mit der Elektrode (1) des Kompartiments 2 hergestellt wird, verläßt der Strom in diesem Kompartiment wieder die Zelle. Der Taster oder Schalter (3a) wird ebenfalls in die Position gestellt, in der ein geschlossener Meßkreis mit dem Kompartiment 2 entsteht, so daß die Spannung entlang der Kanäle (7) über die Kompartimente 2'und 2" bestimmt werden kann. Die Membranabschnitte in den Kompartimenten (K) weisen die Membranspannung der intakten Zelle auf, die normalerweise mit eingestochenen Glasmikroelektroden gemessen werden kann, so daß der Spannungsmeßkreis unmittelbar mit dem in der konventionellen Vorgehensweise verglichen werden kann. Um den Einfluß unterschiedlicher Versuchsbedingungen auf die elektrischen Vorgänge an biologischen Membranen untersuchen zu können, weist die erfindungsgemäße Vorrichtung mindestens 3 Kompartimente auf. Dabei dient ein Kompartiment, welches mit der "anästhetisierenden" Lösung gefüllt wird, zur Depolarisation der Membranspannung und die beiden übrigen Kompartimente der Einstellung der unterschiedlichen Versuchsbedingungen, wie z. B. Zugabe von Agonisten oder Antagonisten von Membrantransportsystemen. Unter dem Begriff "Versuchsbedingungen" soll im Rahmen der vorliegenden Erfindung die Zugabe oder das Füllen der Kompartimente mit unterschiedlichen Substanzen wie z. B. membranaktiven Substanzen, wie Agonisten und Antagonisten von Membrantransportsystemen (Tetraethylammoniumchlorid (TEA); Nifluminsäure (NA); DCCD (Dicyclohexylcarbodiimid); blockierende Ionen wie Co⁺,La³⁺; Tenside) oder aber physikalische Parameter wie z. B. unterschiedliche Temperaturen verstanden werden. Je mehr unterschiedliche Versuchsbedingungen untersucht werden sollen, desto mehr Kompartimente (K) werden erforderlich. Dabei wird die Anzahl der Kompartimente und die Länge des Kanals (5) durch die natürliche Länge der Zellen, die untersucht werden sollen, begrenzt. Die Länge des Kanals (5) sollte in einer vorteilhaften Ausführung der Vorrichtung mindestens die Länge der zu untersuchenden Zelle aufweisen.

Nach Abschluß der Experimente kann die Zelle bei vorsichtiger Entnahme aus der Vorrichtung (aufrechterhaltener Turgor) in künstlichem Teichwasser wieder regeneriert werden.

Die erfindungsgemäße Vorrichtung und das Verfahren sind beispielsweise zur Abwasserüberwachung bzw. zum Biomonitoring geeignet, da auf kleinstem Raum mit einfach handhabbaren biologischen Membranen eine schnelle Analyse von unterschiedlichen membranaktiven Substanzen in einem Analyseschritt durchgeführt werden kann. Anhand von Kalibrationskurven können somit auch qualitative Analysen durchgeführt werden, indem mittels Höhe des Aktionspotentials in Abhängigkeit von der Substanzkonzentration eine Konzentrationsangabe ermittelt werden kann.

### Ausführungsbeispiele:

### 1. Anzucht von Characeen:

Der Boden eines Plastikkübels von etwa 50 L Volumen wurde auf etwa 10 cm Höhe mit autoklavierter Walderde gefüllt. Auf diese wurde autoklavierter Quarzsand in 2-3 cm Höhe geschichtet. Büschel von Characeen-Internodien wurden mit möglichst vielen Nodien in den Sand eingesetzt, nachdem der Anzuchtkübel auf etwa 20 cm Höhe über dem Sand mit Wasser gefüllt wurde. Der Kübel wurde im Verhältnis 2:1 mit deionisiertem Wasser: Leitungswasser aufgefüllt. Nach ca. 4 Wochen beginnen die Algenthalli merklich zu wachsen. Die Wachstumsbedingungen sind gut bei einem Licht/Dunkel-Zyklus von 12/12 h oder 14/10 h bei einer Raumtemperatur von 20-23 °C.

### 2. Präparation einer Internodialzelle:

Internodialzellen werden dem Anzuchtkübel entnommen, die anhängenden Nachbarzellen abgeschnitten und die Zelle über 24 h in künstlichem Teichwasser (s.u.) vorinkubiert. Zum Versuch wird eine Internodialzelle mit Hilfe eines feinen Papiertuchs (Zellstoffpapier möglich) abgetrocknet und in die vorbereitete Meßkammer eingelegt.

### 3. Verwendete Versuchslösungen:

■ Anästhetisierende Lösung in Kompartiment 1: 100 mM KCl
■ Künstliches Teichwasser (artificial pond water = APW) in Kompartimenten 2-7: 0.1 mM KCl, 0.1 mM NaCl, 0.1 mM CaCl₂, 5 mM HEPES/Tris justiert auf pH 7.5, 180 mM Sorbitol
■ Agarbrücken in Kompartimenten 1'-7': 2% w/v Agar in APW
■ Zur Ableitung in den Kompartimenten 1"-7": 1 M KCl

### 4. Gleichzeitige Untersuchung des Einflusses von Tetraethylammoniumchlorid (TEA) und Nifluminsäure (NA) auf das elektrische Verhalten einer Membran von Chara australis

Die Vorrichtung wurde wie oben beschrieben zur Messung vorbereitet und eine Internodialzelle von Chara australis in Kanal (5) eingelegt und mit Vaseline in voneinander unabhängig reagierende Bereiche isoliert. In das Kompartiment 2 wurde neben APW zusätzlich 5 mM Tetraethylammoniumchlorid (TEA) zugegeben, das bekanntlich K⁺-spezifische Ionenkanäle blockiert. In Kompartiment 3 wurde neben APW kein TEA jedoch 50 µM Nifluminsäure (NA) zugegeben, das Cl -spezifische Ionenkanäle blockiert. Fig. 6 zeigt die Unabhängigkeit des elektrischen Verhaltens der Membranabschnitte in den jeweiligen Kompartimenten, in die die unterschiedlichen membranaktiven Substanzen eingefüllt wurden. Im Verlauf des elektrisch hervorgerufenen Aktionspotentials in Kompartiment 2 erkennt man deutlich die verlangsamte Repolarisation (vgl. Abb. 3), die durch den - hier behinderten-Ausstrom von K⁺ erzeugt wurde, während im Kompartiment 3 die Anwesenheit der Nifluminsäure die Entstehung eines Aktionspotentials nahezu komplett unterband; die Depolarisationsphase des Aktionspotentials wird durch einen Cl⁻-Ausstrom aus der Zelle erzeugt. Eine wiederholte anschließende Messung in Kompartiment 2 (APW-TEA) ergab den identischen Verlauf des Aktionspotentials wie zu Beginn. Diese Experimente an einer Zelle zeigen, daß die partitionierten Membranflächen unabhängig voneinander reagieren.

## Patentansprüche

1. Vorrichtung zur simultanen Messung unterschiedlicher Versuchsparameter auf das elektrische Verhalten einer biologischen Membran einer einzigen Zelle, **gekennzeichnet durch**
- eine Basisplatte (B) mit mindestens 3 Kompartimenten (K), an die jeweils eine Elektrode (1) zur Stromzufuhr angeschlossen ist,
- wobei die Kompartimente (K) sowohl über einen Kanal (5) in einer Reihe X₁ miteinander verbunden sind, als auch jeweils über einen zweiten Kanal (7) mit weiteren Kompartimenten (K', K") verbunden sind,
- und die Kompartimente (K") jeweils an eine Elektrode (1a) zur Ableitung von Spannung angeschlossen sind,
- und wobei die jeweiligen Kompartimente (K) untereinander elektrisch isoliert sind, indem sich im Kanal (5) jeweils zwischen den Kompartimenten (K) ein elektrisch isolierendes Mittel befindet,
**dadurch gekennzeichnet, dass**
jedes Kompartiment K der Reihe X₁ über den zweiten Kanal (7) mit nur einem Kompartiment K' der Reihe X₂ und dieses wiederum mit nur einem Kompartiment K" der Reihe X₃ verbunden ist, und dass
sowohl die Kompartimente K' der Reihe X₂ als auch die Kompartimente K" der Reihe X₃ nicht miteinander verbunden sind.

2. Vorrichtung nach Anspruch 1,
**dadurch gekennzeichnet, daß**
die Länge des Kanals (5) mindestens der Länge der zu untersuchenden Zelle entspricht.

3. Vorrichtung nach einem der Ansprüche 1 bis 2,
**dadurch gekennzeichnet, daß**
die Stromzufuhr über die Elektroden (1) bzw. die Ableitung der Spannung über die Elektroden (1a) für jedes Kompartiment (K) bzw. (K") einzeln gesteuert ist.

4. Vorrichtung nach einem der Ansprüche 1 bis 3,
**dadurch gekennzeichnet, daß**
die Stromzufuhr bzw. Ableitung der Spannung der
Elektroden (1, 1a) über Taster oder Schalter (3, 3a) gesteuert ist.

5. Vorrichtung nach einem der Ansprüche 1 bis 4,
**dadurch gekennzeichnet, daß**
mindestens ein Kompartiment (K) mit einer Substanz gefüllt ist, die zur Depolarisation der Zellmembran führt, und die weiteren Kompartimente (K) eine der Zelle physiologisch angepaßte Substanz enthält, die membranaktive Substanzen enthält oder in die diese membranaktiven Substanzen zugegeben sind.

6. Vorrichtung nach einem der Ansprüche 1 bis 5,
**dadurch gekennzeichnet, daß**
die Kompartimente (K') mit einer diffusionshemmenden Substanz gefüllt sind.

7. Vorrichtung nach einem der Ansprüche 1 bis 6,
**dadurch gekennzeichnet, daß**
die Kompartimente (K") mit einer Lösung hoher Ionenstärke gefüllt sind, die unterschiedliche Elektrodenpotentiale verhindert.

8. Verfahren zur simultanen Messung unterschiedlicher Versuchsparameter auf das elektrische Verhalten einer biologischen Membran einer einzigen Zelle bei dem eine Vorrichtung gemäß einem der Ansprüche 1 bis 7 eingesetzt wird,
wobei die zu untersuchende Zelle so eingebracht wird, dass sie sich in mindestens 3 Kompartimenten (K) erstreckt, eins der Kompartimente (K) mit einer Substanz gefüllt wird, die die Membran der zu untersuchenden Zelle in diesem Kompartiment (K) depolarisiert, und wobei die jeweiligen Kompartimente (K) untereinander elektrisch isoliert werden, indem in den Kanal (5) jeweils zwischen den Kompartimenten (K) ein elektrisch isolierendes Mittel gefüllt wird, die Membranbereiche in den Kompartimenten gleichzeitig unterschiedlichen Versuchsbedingungen ausgesetzt und die elektrischen Signale der jeweiligen Membranbereiche ausgewertet werden.

9. Verfahren nach Anspruch 8,
**dadurch gekennzeichnet, daß** die Zelle durch ein
Isolationsmittel in mindestens 3 unabhängig voneinander reagierende Bereiche geteilt wird.

10. Verfahren nach Anspruch 9,
**dadurch gekennzeichnet, daß**
das Isolationsmittel Vaseline ist.

11. Verfahren nach einem der Ansprüche 8 bis 10,
**dadurch gekennzeichnet, daß**
an die unabhängig voneinander reagierenden Bereiche der Zelle separat ein Strom angelegt wird und separat die Membranspannung dieses Bereichs ausgewertet wird.

12. Verfahren nach einem der Ansprüche 8 bis 11,
**dadurch gekennzeichnet, daß**
die unabhängig voneinander reagierenden Bereiche der Zelle jeweils in ein Kompartiment (K) eingelegt werden, welches neben einer, der Zelle physiologisch angepaßten Substanz, membranaktive Substanzen enthält oder in welches diese membranaktiven Substanzen zugegeben werden.

13. Verfahren nach vorhergehendem Anspruch,
**dadurch gekennzeichnet, daß**
als membranaktive Substanzen Agonisten oder Antagonisten von Membrantransportsystemen eingesetzt werden.

14. Verfahren nach einem der Ansprüche 8 bis 13,
**dadurch gekennzeichnet, daß**
jedes Kompartiment (K) unterschiedliche membranaktive Substanz enthält bzw. in jedes eine unterschiedliche membranaktive Substanz zugegeben wird.

15. Verfahren nach einem der Ansprüche 8 bis 14,
**dadurch gekennzeichnet, daß**
langgestreckte pflanzliche oder tierische Zellen eingesetzt werden.

16. Verfahren nach einem der Ansprüche 8 bis 15,
**dadurch gekennzeichnet,**
**daß** Zellen aus der Gruppe der Charophyten eingesetzt werden.

17. Verfahren nach einem der Ansprüche 8 bis 16,
**dadurch gekennzeichnet,**
**daß** Zellen der Gattung Chara australis oder Chara corallina eingesetzt werden.

## Claims

1. Apparatus for simultaneous measurement of different test parameters regarding the electrical behaviour of a biological membrane of single cell, **characterised by**
- a base plate (B) with at least 3 compartments (K), to each of which an electrode (1) is connected for the current supply,
- in which the compartments (K) are connected to each other through channel (5) in a row X₁ and with further compartments (K', K") through a second channel (7),
- and the compartments (K") are each connected to an electrode (1a) to draw off the voltage,
- and in which the relevant compartments (K) are electrically isolated from each other, in that there is a means of electrical isolation in the channel (5) between each of the compartments (K),
**characterised in that**
each compartment K of the row X₁ is connected to only one compartment K' of the row X₂ through the second channel (7) and in turn this is connected to only one compartment K" of the row X₃, and that
the compartments K' of the row X₂ and the compartments K" of the row X₃ are not connected to each other.

2. Apparatus according to claim 1.
**characterised in that**
the length of the channel (5) corresponds to at least the length of the cell to be examined.

3. Apparatus according to one of claims 1 to 2,
**characterised in that**
the current supply is controlled individually for each compartment (K) or (K") through the electrodes (I) or drawing off the voltage through the electrodes (1a).

4. Apparatus according to one of claims 1 to 3,
**characterised in that**
the current supply or drawing off the voltage from the electrodes (1, 1a) is controlled through keys or switches (3, 3a).

5. Apparatus according to one of claims 1 to 4,
**characterised in that**
at least one compartment (K) is filled with a substance, which leads to depolarisation of the cell membrane, and the other compartments (K) contain a substance physiologically adapted to the cells, which contains membrane active substances or to which these membrane active substances are added.

6. Apparatus according to one of claims 1 to 5,
**characterised in that**
the compartments (K') are filled with a diffusion inhibiting substance.

7. Apparatus according to one of claims 1 to 6,
**characterised in that**
the compartments (K") are filled with a solution with a high ionic strength, which prevents different electrode potentials.

8. Method for simultaneous measurement of different test parameters regarding the electrical behaviour of a biological membrane of a single cell, in which a device according to one of claims 1 to 7 is used,
in which the cell to be examined is introduced so that the it extends into at least 3 compartments (K), one of the compartments (K) is filled with a substance, which depolarises the membrane of the cell to be examined in this compartment (K), and in which the relevant compartments (K) are electrically isolated from each other, in which a means of electrical isolation is filled into the channel (5) between the compartments (K), which subjects the membrane areas in the compartment immediately to different test conditions and the electrical signals of the relevant membrane areas are evaluated.

9. Method according to claim 8,
**characterised in that** the cell is divided by a
means of isolation into at least 3 areas reacting independently of each other.

10. Method according to claim 9,
**characterised in that**
the means of isolation is vaseline.

11. Method according to claim 8 to 10.
**characterised in that**
a current is applied separately to the areas of the cell reacting independently of each other and the membrane voltage of this area is evaluated separately.

12. Method according to claim 8 to 11,
**characterised in that**
the areas of the cells reacting independently of each other are put in a compartment (K), which as well as a substance physiologically adapted to the cell contains membrane active substances or to which these membrane active substances are added.

13. Method according to the previous claim,
**characterised in that**
agonists or antagonists of membrane transport systems are used as membrane active substances.

14. Method according to one of claims 8 to 13,
**characterised in that**
each compartment (K) contains different membrane active substances or a different membrane active substance is added to each of them.

15. Method according to one of claims 8 to 14.
**characterised in that**
elongated plant or animal cells are used.

16. Method according to one of claims 8 to 15,
**characterised in that**,
cells from the charophytes group are used.

17. Method according to one of claims 8 to 15,
**characterised in that**,
cells of the Chara australis or Chara corallina genus are used.

## Revendications

1. Appareil de mesure simultanée de différents paramètres d'essai relativement au comportement électrique d'une membrane biologique d'une unique cellule, **caractérisé par**
- une plaque de base (B) comprenant au moins 3 compartiments (K), auxquels est connectée respectivement une électrode (1) pour l'alimentation électrique,
- les compartiments (K) étant reliés aussi bien les uns aux autres par le biais d'un canal (5) en une ligne X₁ que respectivement avec d'autres compartiments (K', K") par le biais d'un deuxième canal (7),
- et les compartiments (K") étant reliés respectivement à une électrode (1a) pour la dérivation de la tension,
- et les compartiments (K) respectifs étant isolés électriquement les uns vis-à-vis des autres du fait qu'un milieu électriquement isolant se trouve dans le canal (5) respectivement entre les compartiments (K),
**caractérisé en ce que**
chaque compartiment K de la ligne X₁ est relié par le biais du deuxième canal (7) à seulement un compartiment K' de la ligne X₂ et celui-ci est de nouveau relié à seulement un compartiment K" de la ligne X₃, et **en ce que**
aussi bien les compartiments K' de la ligne X₂ que les compartiments K" de la ligne X₃ ne sont pas reliés entre eux.

2. Appareil selon la revendication 1,
**caractérisé en ce que**
la longueur du canal (5) correspond au moins à la longueur de la cellule à analyser.

3. Appareil selon l'une quelconque des revendications 1 à 2,
**caractérisé en ce que**
l'alimentation électrique est commandée par le biais des électrodes (1) ou la dérivation de la tension est commandée par le biais des électrodes (1a) individuellement pour chaque compartiment (K) ou (K').

4. Appareil selon l'une quelconque des revendications 1 à 3,
**caractérisé en ce que**
l'alimentation électrique ou la dérivation de la tension des électrodes (1, 1a) est commandée par le biais d'un bouton ou d'un interrupteur (3, 3a).

5. Appareil selon l'une quelconque des revendications 1 à 4,
**caractérisé en ce que**
au moins un compartiment (K) est rempli d'une substance qui provoque la dépolarisation de la membrane cellulaire, et les autres compartiments (K) contiennent une substance adaptée physiologiquement à la cellule, qui comprend des substances actives sur la membrane ou à laquelle ces substances actives sur la membrane sont additionnées.

6. Appareil selon l'une quelconque des revendications 1 à 5,
**caractérisé en ce que**
les compartiments (K') sont remplis d'une substance inhibant la diffusion.

7. Appareil selon l'une quelconque des revendications 1 à 6,
**caractérisé en ce que**
les compartiments (K") sont remplis d'une solution à forte puissance ionique, qui empêche des potentiels d'électrodes différents.

8. Procédé de mesure simultanée de différents paramètres d'essai relativement au comportement électrique d'une membrane biologique d'une unique cellule, dans lequel un appareil selon l'une quelconque des revendications 1 à 7 est mis en oeuvre,
la cellule à analyser étant placée de sorte qu'elle s'étende au moins sur 3 compartiments (K), un des compartiments (K) étant rempli d'une substance qui dépolarise la membrane de la cellule à analyser dans ce compartiment (K), et les compartiments (K) respectifs étant isolés électriquement les uns vis-à-vis des autres du fait qu'un milieu électriquement isolant est chargé dans le canal (5) respectivement entre les compartiments (K), les zones de la membrane dans les compartiments étant soumises simultanément à des conditions d'essai différentes et les signaux électriques des zones respectives de la membrane étant évalués.

9. Procédé selon la revendication 8,
**caractérisé en ce que** la cellule est divisée par un
milieu d'isolation en au moins 3 zones réagissant de manière indépendante les unes des autres.

10. Procédé selon la revendication 9,
**caractérisé en ce que**
le milieu d'isolation est la vaseline.

11. Procédé selon l'une quelconque des revendications 8 à 10,
**caractérisé en ce que**
un courant est appliqué séparément aux zones de la cellule réagissant de manière indépendante les unes des autres et la tension de la membrane de cette zone est évaluée séparément.

12. Procédé selon l'une quelconque des revendications 8 à 11,
**caractérisé en ce que**
les zones de la cellule réagissant de manière indépendante les unes des autres sont insérées respectivement dans un compartiment (K) qui contient, en plus d'une substance adaptée physiologiquement à la cellule, des substances actives sur la membrane ou auquel ces substances actives sur la membrane sont additionnées.

13. Procédé selon la revendication précédente,
**caractérisé en ce que**
l'on met en oeuvre à titre de substances actives sur la membrane des agonistes ou antagonistes de systèmes de transport membranaire.

14. Procédé selon l'une quelconque des revendications 8 à 13,
**caractérisé en ce que**
chaque compartiment (K) comprend une substance active sur la membrane différente ou dans chacun d'eux, une substance active sur la membrane différente est additionnée.

15. Procédé selon l'une quelconque des revendications 8 à 14,
**caractérisé en ce que**
l'on met en oeuvre des cellules allongées végétales ou animales.

16. Procédé selon l'une quelconque des revendications 8 à 15,
**caractérisé en ce**
**que** l'on met en oeuvre des cellules provenant du groupe des charophytes.

17. Procédé selon l'une quelconque des revendications 8 à 16,
**caractérisé en ce**
**que** l'on met en oeuvre des cellules du genre *Chara australis* ou *Chara corallina.*
